# EUROPÄISCHE PATENTANMELDUNG

(11) **EP 2 647 649 A1**
(43) Veröffentlichungstag der Anmeldung: **09.10.2013**
(21) Anmeldenummer: 12002457.5
(22) Anmeldetag: 04.04.2012
(51) Int. Cl.: C08B 37/00, C08L 5/08, A01N 59/20, A61K 31/728, A61K 33/34, A61K 33/38

(54) **Hyaluronsäuregel, Verfahren zur Herstellung des Hyaluronsäuregels, und Medizinprodukt mit dem Hyaluronsäuregel**

(71) Anmelder: Witt, Andreas, 34305 Niedenstein (DE)
(72) Erfinder: Witt, Andreas, 34305 Niedenstein (DE)
(74) Vertreter: Walkenhorst, Andreas

(57) **Zusammenfassung**

Ein Hyaluronsäuregel (1) auf der Basis quervernetzter Hyaluronsäure soll angegeben werden, mit der die günstigen Eigenschaften dieses Materials für eine Anzahl noch weiterer medizinischer und/oder therapeutischer Anwendungen nutzbar gemacht werden können. Dazu weist das Hyaluronsäuregel (1) erfindungsgemäß aus quervernetzter Hyaluronsäure gebildete Gelpartikel (2) auf, in die Partikel (4) eines bioziden Metalls eingebettet sind.

## Beschreibung

Die Erfindung betrifft ein Hyaluronsäuregel mit aus quervernetzter Hyaluronsäure gebildeten Gelpartikeln. Sie bezieht sich weiter auf ein Verfahren zur Herstellung des Hyaluronsäuregels sowie auf die Verwendung des Hyaluronsäuregels in kosmetischen Mitteln oder Medizinprodukten.

Hyaluronsäure ist als körpereigener Stoff biokompatibel und nicht immunogen und weist im Hinblick auf zahlreiche Körperfunktionen besonders günstige Eigenschaften wie beispielsweise eine hohe Fähigkeit zur Wasserspeicherung, hohe Druckbeständigkeit (insbesondere aufgrund des eingelagerten Wassers) sowie günstige Viskosität, die sie als körpereigenes Schmiermittel für Gelenke einsetzbar macht, auf. Aus diesen Gründen findet Hyaluronsäure vielfältige medizinische Anwendungen, wie beispielsweise bei der Wundheilung, in der Orthopädie und in der Augenheilkunde. Ebenfalls wird Hyaluronsäure in Kosmetika verwendet.

Hyaluronsäure gehört zur Klasse der Glykosaminoglykane und besteht aus einem langkettigen linearen Polysaccharid. Hierbei handelt es sich insbesondere um ein lineares Kettenmolekül mit repetitiven Disaccharid-Einheiten (N-Acetylglucosamin-Glucuronsäure). Hyaluronsäure kommt für gewöhnlich in Form des entsprechenden Natriumsalzes mit der Formel [C₁₄H₂₀NNaO₁₁]ₙ vor, wobei n von der Herkunft der Hyaluronsäure und der Isolationsmethode abhängt. Sie kann aus einer Vielzahl von Quellen isoliert werden wie beispielsweise aus der menschlichen Nabelschnur, Hahnenkämmen und dem Bindegewebe von Wirbeltieren. Die Säure ist ebenfalls ein Bestandteil von Bakterien wie Streptokokken und lässt sich daher auch durch Fermentationsprozesse erhalten.

Bei der Nutzung in Präparaten zu therapeutischen oder kosmetischen Zwecken spielt insbesondere die Haltbarkeit nach der Einbringung in den menschlichen Körper eine Rolle. In ersten Entwicklungsstufen wurden aviär hergestellte Hyaluronsäurepräparate, also vergleichsweise langkettige Hyaluronsäuremoleküle aus dem Hahnenkamm, eingesetzt, die durch Quervernetzung mit Vinylsulfon haltbarer gemacht wurden als die native, nicht vernetzte Hyaluronsäure. Diese langkettigen Moleküle mussten dann, um sie mit einer feinen Nadel in die Haut injizieren zu können, in Mikropartikel zerkleinert und in ein Lubricans von unvernetzter Hyaluronsäure eingebracht werden. So entstanden so genannte biphasische Produkte.

In folgenden Entwicklungsstufen wurde Hyaluronsäure bakteriell-fermentativ gewonnen (insbesondere über Streptokokken) und mittels eines geeigneten Vernetzungsmittels, vorzugsweise BDDE (1,4 Butandiol-Diglycidylether), quervernetzt und damit stabiler gemacht. Die Quervernetzung wird dabei durch den Anteil der Sulfonbrücken zwischen den Molekülketten bestimmt. Auch diese Hyaluronsäure musste, um injizierbar zu werden, fragmentiert und mit unvernetzter, nur kurz haltbarer Hyaluronsäure gemischt werden. Die Entwicklung schritt weiter voran und biphasische Hyaluronsäurepräparate wurden mithilfe der so genannten Doppelquervernetzung versucht haltbarer zu machen, wobei sequentiell zwei aufeinanderfolgende Behandlungsschritte zur Quervernetzung eingesetzt werden.

Der Erfindung liegt die Aufgabe zugrunde, ein Hyaluronsäuregel auf der Basis quervernetzter Hyaluronsäure anzugeben, mit der die günstigen Eigenschaften dieses Materials für eine Anzahl noch weiterer medizinischer und/oder therapeutischer Anwendungen nutzbar gemacht werden können. Des Weiteren sollen ein zur Herstellung des Hyaluronsäuregels geeignetes Verfahren sowie eine besonders günstige Verwendung für das Hyaluronsäuregel angegeben werden.

Bezüglich des Hyaluronsäuregels wird diese Aufgabe erfindungsgemäß gelöst mit aus quervernetzter Hyaluronsäure gebildeten Gelpartikeln, in die Partikel eines bioziden Metalls eingebettet sind.

Die Erfindung geht dabei von der Erkenntnis aus, dass gerade die quervernetzte Hyaluronsäure Eigenschaften aufweist, die sie als geeignetes Depotmaterial zur gezielt verlangsamten Abgabe von Wirkstoffen in den (menschlichen) Körper über einen längeren Zeitraum hinweg nutzbar machen. Bei der Injektion der Hyaluronsäure in menschliches Gewebe bzw. in Wundareal wird diese nämlich im Wesentlichen in zwei Stufen abgebaut. In einer ersten Stufe werden durch Hydrolyse die Esterbindungen zwischen den Hyaluronsäureketten aufgespalten. In der zweiten Stufe splaten die so genannten Hyaluronidasen die Hyaluronsäureketten in Fragmente, die dann ihrerseits wiederum zur Biostimulation freigesetzt werden. Dieser Prozess, der bei unvernetzter Hyaluronsäure in vergleichsweise kurzen Zeiträumen von etwa einigen Tagen abläuft, nach denen die Hyaluronsäure rückstandslos abgebaut wurde, kann durch die Quervernetzung verlangsamt werden, so dass bei quervernetzter Säure ein vollständiger Abbau im Körper über einen Zeitraum von bis zu einem Jahr erstreckt werden kann.

Dies soll nun zur gezielten dosierten Abgabe von Wirkstoffen in den Körper in der Art eines Depots genutzt werden, indem die Wirkstoffe geeignet in der Art von Kapselbildung in aus quervernetzter Hyaluronsäure gebildete Gelpartikel eingebettet werden. Beim Abbau der Hyaluronsäure im Körper löst sich dann die durch die quervernetzte Hyaluronsäure gebildete äußere Hülle diese Kapseln oder Gelpartikel zu gegebenem Zeitpunkt auf, so dass eine Abgabe des zuvor im Inneren der Kapsel oder des Gelpartikels eingeschlossenen Wirkstoffs erfolgt. Im Gegensatz zu Konzepten, bei denen Wirkstoffe an Hyaluronsäure-Moleküle gebunden und über diese beispielsweise an einen Zielort transportiert werden, bewirkt die nun vorgesehene Einbettung, dass aufgrund der die Metallpartikel vollständig umgebenden Umhüllung der Gelpartikel ein Kontakt der Metallpartikel mit der Umgebung nicht stattfinden kann, bis die Umhüllung durch Abbau der Hyaluronsäure aufgelöst ist. Damit wird die Freisetzung der eingebetteten Partikel und damit der Beginn von deren Wirksamkeit auf den genannten Zeitpunkt aufgeschoben.

Dieses eigenständig als erfinderisch angesehene Grundkonzept soll nun gezielt für die dosierte Einbringung eines bioziden, also keimtötenden und somit desinfizierenden, Wirkstoffs, vorliegend eines bioziden Metalls, in die jeweilige Körperregion genutzt werden. Damit ist ein Hyaluronsäuregel bereitstellbar, das insbesondere als Behandlungs- oder Heilmittel für Wunden genutzt werden kann. Durch die über einen vergleichsweise langen Zeitraum erstreckte Abgabe des ursprünglich in die Gelpartikel eingebetteten bioziden Wirkstoffs kann nämlich über einen entsprechend langen Zeitraum hinweg die gezielte Beaufschlagung des Wundenbereichs mit keimtötenden Substanzen aufrechterhalten werden, so dass Entzündungen und dergleichen dauerhaft wirksam bekämpft und der Heilungsprozess somit über den gesamten Zeitraum hinweg begünstigt und unterstützt werden kann. Die Verwendung von biozidem Metall als eingebetteter Wirkstoff ist hierbei besonders günstig, da dieses Metall bei ausreichend hoher biozider Wirksamkeit in geeigneter, in die Gelpartikel integrierbarer Partikelgröße bereitgestellt werden kann.

Als biozides Metall zur Bildung der in die Gelpartikel eingebetteten Partikel ist dabei in vorteilhafter Ausgestaltung Kupfer oder ganz besonders bevorzugt Silber vorgesehen. Gerade für diese Metalle ist die biozide Wirksamkeit auch bei vergleichsweise kleiner Partikelgröße besonders zuverlässig gegeben, so dass eine besonders geeignete Kombination mit den aus quervernetzter Hyaluronsäure gebildeten Gelpartikeln bereitstellbar ist.

In besonders vorteilhafter Ausgestaltung weisen die Partikel des bioziden Metalls eine mittlere Partikelgröße von etwa 0,2 µm bis 20 µm, vorzugsweise von etwa 10 µm, auf. Insbesondere kommt auch die Verwendung von kolloidalem Silber als Wirkstoff in Betracht, von dessen Wirksamkeit bei der Infektionsbekämpfung man seit geraumer Zeit ausgeht.

Die aus quervernetzter Hyaluronsäure gebildeten Gelpartikel sind besonders vorteilhaft gezielt für den vorgesehenen Einsatzzweck, nämlich die vorübergehende Bildung einer Umhüllung oder Kapsel für die eingebetteten Metallpartikel, ausgestaltet. Dazu weist vorteilhafterweise die die Gelpartikel bildende Hyaluronsäure einen Vernetzungsgrad (gemäß üblicher Definition) von höchstens 65 %, bevorzugt von höchstens 10 %, auf. In alternativer oder zusätzlicher vorteilhafter Weiterbildung weisen die Gelpartikel eine mittlere Partikelgröße von etwa 50 µm bis etwa 600 µm, vorzugsweise von etwa 150 µm bis höchstens 300 µm, auf. Gerade in Kombination mit der bevorzugten Partikelgröße für die eingebetteten bioziden Metallpartikel ist somit der die Hülle bildende Gelpartikel etwa um den Faktor 10 bis 15 größer als der eingebettete Metallpartikel. Damit ist bis zur Auflösung der durch den Gelpartikel gebildeten Umhüllung aufgrund der Abbauprozesse der Hyaluronsäure eine wirksame Verkapselung der eingebetteten Metallpartikel sichergestellt. Andererseits ist durch die vorgesehene bevorzugte Begrenzung der Größe der Gelpartikel sichergestellt, dass das Hyaluronsäuregel vergleichsweise einfach auch unter Rückgriff auf Kanülen mit therapeutisch akzeptabler Größe appliziert werden kann, ohne dass in zu großem Maße mit einer Verblockung oder Verstopfung gerechnet werden muss.

Das Hyaluronsäuregel gemäß der Erfindung könnte als so genanntes mono- oder einphasiges Gel ausgeführt sein. In besonders vorteilhafter Ausgestaltung ist das Gel aber mehrkomponentig oder mehrphasig, insbesondere als biphasisches Gel, ausgeführt. Dabei ist besonders bevorzugt zusätzlich zu einem ersten Gelanteil, der die die Partikel aus biozidem Metall enthaltenden Gelpartikel umfasst, noch zumindest ein zweiter Gelanteil, umfassend unvernetzte Hyaluronsäure, vorgesehen. Durch diese besonders bevorzugte Ausführung kann das Hyaluronsäuregel gezielt für besonders günstige Eigenschaften gerade bei einem Einsatz zur Wundbehandlung oder dergleichen ausgestaltet sein. Der erste, die Gelpartikel enthaltende Gelanteil ist dabei gezielt zur verzögerten oder retardierten, dosierten Abgabe der eingebetteten Metallpartikel und damit zur Bekämpfung oder Unterdrückung von Infektionen ausgeführt. Allerdings muss dabei in Kauf genommen werden, dass die hierfür verwendete Hyaluronsäure aufgrund ihrer Vernetzung nicht oder nur begrenzt heilend oder begünstigend für den Knochen- oder Gewebeaufbau wirkt. Um dennoch besonders günstige Wundheileigenschaften durch das Gel zu erreichen, umfasst dieses bevorzugt, insbesondere in der Art eines biphasischen oder Hybrid-Aufbaus, den zweiten Anteil aus unvernetzter Hyaluronsäure, die aufgrund ihrer bekannten Eigenschaften begünstigend für den Knochen- oder Gewebeaufbau wirkt.

Das biozide Metall, insbesondere Silber, liegt in dem Hyaluronsäuregel vorteilhafterweise mit einem Gewichtsanteil von 0,1 % bis 0,5 %, vorzugsweise von höchstens 0,3 %, ganz besonders bevorzugt von etwa 0,3 %, vor. Damit ist sichergestellt, dass die gewünschten bakterioziden Eigenschaften des Silberanteils zuverlässig genutzt werden können, ohne dass infolge eines zu hohen Silberanteils eine Schädigung von Körperzellen oder -gewebe in Kauf genommen werden müsste.

Bezüglich des Verfahren zur Herstellung eines Hyaluronsäuregels, insbesondere der vorgenannten Art, wird die genannte Aufgabe gelöst, indem
- Hyaluronsäure mit Partikeln eines bioziden Metalls versetzt wird, so dass sich eine Suspension bildet, und
- der Suspension ein Vernetzungsmittel für die Hyaluronsäure zugegeben wird, so dass die Hyaluronsäure quervernetzt wird.

Hierbei ist insbesondere vorgesehen, dass der Prozess der Quervernetzung der Hyaluronsäure - ausgelöst durch Zugabe des Vernetzungsmittels - erst angestoßen wird, nachdem eine Suspension hergestellt wurde, in der die Metallpartikel in der Hyaluronsäure in Schwebe gehalten sind. Nach sorgfältiger Durchmischung dieser Komponenten, die eine weitgehend gleichmäßige Verteilung der bioziden Metallpartikel in der Ausgangslösung der Hyaluronsäure bewirkt, wird die Quervernetzung in Gang gesetzt. Beim Prozess der Quervernetzung erfolgt somit der Einschluss der Metallpartikel in die sich infolge der Quervernetzung bildenden Gelpartikel.

Vorteilhafterweise wird dazu zunächst ein Vordispergieren der Metallpartikel in einem geeigneten Dispergiermittel, für Silber beispielsweise Glycerin, vorgenommen. Die dabei entstehende Dispersion oder Suspension des Metalls (insbesondere Silbers) im Dispergiermittel (insbesondere Glycerin) wird anschließend vorzugsweise kontinuierlich in Bewegung gehalten, z. B. durch Rühren, so dass ein Sedimentieren vermieden werden kann. Diese Suspension oder Dispersion wird sodann in geeigneten Mengenverhältnissen mit der Ausgangslösung der Hyaluronsäure vermischt, so dass die gewünschte Suspension der Metallpartikel in der Hyaluronsäure entsteht. Nachdem wie vorstehend beschrieben die Quervernetzung der Hyaluronsäure mit Einschluss der Metallpartikel in die entstehenden Gelpartikel durchgeführt wurde, wird in zusätzlicher vorteilhafter Weiterbildung ein biphasisches Gel hergestellt, indem als weitere Komponente noch unvernetzte Hyaluronsäure zugemischt wird.

In besonders vorteilhafter Ausgestaltung wird durch geeignete Prozessführung, also insbesondere durch eine geeignete Wahl von Reaktionszeiten und -parametern wie beispielsweise Druck, Temperatur, Mischungsverhältnissen und dergleichen, die Vernetzung der Hyaluronsäure derart durchgeführt, dass sich ein Vernetzungsgrad von höchstens 10 % einstellt.

Aufgrund des an sich möglicherweise unerwünschten Einflusses der Quervernetzung auf die Biokompatibilität, die hierdurch beeinträchtigt werden kann, können auch nur kleinste Mengen an Quervernetzungen (vorzugsweise etwa 1 %) eingesetzt werden. Trotz derartig geringer Mengen an Quervernetzungen wird eine weitgehende Stabilisierung bei nur geringfügiger Modifikation (max 1 %) des Hyaluronsäure-Moleküls erreicht. Die Stabilisierung basiert dabei auf einer minimalen Bindung zwischen benachbarten Hyaluronsäuremolekülen. Dadurch entsteht bei derartig gering gehaltenen Vernetzungsgraden eine dreidimensionale Gelmatrix, aus der Gelpartikel der für die gewünschte Kapselbildung geeigneten Größen hergestellt. Durch die hierdurch erreichte so genannte "sanfte Stabilisierung" der Hyaluronsäure wird ihre Halbwertzeit in der Haut um ein vielfaches von nur wenigen Tagen bis zu einigen Monaten, in einigen Fällen bis zu Jahren erhöht, so dass die gewünschte verzögerte und zeitlich erstreckte Abgabe der eingebetteten Partikel in das umgebende Gewebe besonders günstig erreicht werden kann.

Nach den genannten Verfahrensschritten wird das erhaltene Zwischenprodukt mit den entstandenen Partikeln aus quervernetzter Hyaluronsäure mit darin eingebetteten Metallpartikeln vorteilhafterweise an sich üblichen Schritten der weiteren Behandlung unterzogen. Vorteilhafterweise erfolgt ein Quellvorgang, und/oder das nach der Vernetzung erhaltene Hyaluronsäuregel wird gefiltert und gewaschen. Dabei wird insbesondere in vorteilhafter Weiterbildung dem Umstand und dem Auslegungsziel Rechnung getragen, dass bei eventuell zu befürchtenden toxischen Wirkungen des Vernetzungsmittels dessen nicht bei der Vernetzung umgesetzte Reste vollständig aus dem hergestellten Hyaluronsäuregel ausgewaschen und entfernt werden.

Die Vernetzung kann durch Zugabe eines Vernetzungsmittels ausgelöst und durchgeführt werden. Als Vernetzungsmittel kann dabei grundsätzlich eines der für die Quervernetzung von Hyaluronsäure gängigen Mittel verwendet werden. Vorteilhafterweise wird als Vernetzungsmittel 1,2-Bis(2,3-Epoxypropoxy)ethylen oder 1-(2,3-Epoxypropyl)-2,3-epoxycyclohexan, in ganz besonders bevorzugter Ausgestaltung 1,4-Butandioldiglycidylether (BDDE) verwendet. In alternativer oder zusätzlicher vorteilhafter Ausgestaltung wird die Quervernetzung unterstützend zum Vernetzungsmittel oder auch anstelle der Zugabe eines Vernetzungsmittels als so genannte "photo cross linking"-Vernetzung, also als UV-Licht-induzierte Quervernetzung, durchgeführt, indem die Suspension aus Hyaluronsäure und darin in Schwebe gehaltenen Metallpartikeln mit UV-Licht, vorzugsweise einer im Hinblick auf die Vernetzungsprozesse geeignet gewählten Wellenlänge, bestrahlt wird.

Vorteilhafterweise weist die eingesetzte Hyaluronsäure ein Molekulargewicht von 300.000 bis 2.500.000, bevorzugt von 400.000 bis 1.500.000, besonders bevorzugt von 500.000 bis 1.100.000, atomaren Masseneinheiten auf.

Alternativ zur oben genannten Lösung wird die genannte Aufgabe bezüglich des Hyaluronsäuregels gelöst durch ein Hyaluronsäuregel, erhältlich durch vorstehend beschriebene Verfahren.

Das Hyaluronsäuregel, charakterisiert durch die genannten Eigenschaften und/oder erhältlich durch das genannte Verfahren, wird insbesondere und gemäß einem eigenständig erfinderischen Gedanken verwendet in einem kosmetischen oder medizinischen Mittel, insbesondere in einem Medizinprodukt oder einem Medikament.

Ebenfalls ein Gegenstand der vorliegenden Erfindung ist ein Medizinprodukt enthaltend ein Hyaluronsäuregel der oben genannten Art bzw. erhältlich nach dem vorstehend beschriebenem Verfahren und gegebenenfalls ein oder mehrere pharmazeutische Hilfsmittel. Diese können beispielsweise sein: Lösungsmittel, beispielsweise wässrige Pufferlösung, Lösungsvermittler, Lösungsbeschleuniger, Salze, Salzbildner, Puffersubstanzen, Viskositätsregler, Konsistenzbeeinflusser wie Verdickungsmittel, Sedimentationsverzögerer, Antioxidantien, Konservierungsmittel, Mittel zur Isotonisierung, Tenside, Emulgatoren, Solubilisatoren, Benetzer, Entschäumer, Spreitmittel, Geruchs- und Geschmackskorrigentien, Resorptionsbeschleuniger.

Vorteilhafterweise weist das Hyaluronsäuregel eine für den vorgesehenen Anwendungszweck geeignet gewählte Viskosität auf. Hierzu kann bedarfsweise bei oder nach der Herstellung des Hyaluronsäuregels ein Viskositätsregler zugegeben werden. Ein Viskositätsregler zur Einstellung einer gewünschten Viskosität kann vorzugsweise aus der Gruppe bestehend aus Chondroitinsulfat, Polyvinylpyrrolidon, Polyvinylalkohol, Polyacrylsäure, Polyacrylamid, Polyacrylharze, Polyethylenglykol, Cellulosederivate sowie Mischungen davon ausgewählt werden. Als geeignete Cellulosederivate können beispielsweise Hydroxyethylcellulose, Hydroxypropylmethylcellulose und Carboxymethylcellulose verwendet werden.

Bevorzugt eignet sich das erfindungsgemäße Medizinprodukt zur Schleimhaut- oder Wundbehandlung, bevorzugt zur Wundheilung. In alternativer bevorzugter Ausgestaltung ist das Medizinprodukt zur Behandlung und/oder Prophylaxe von Parodontose oder Periimplantitis, insbesondere im Zusammenhang mit der Insertion und/oder Nachsorge von Implantaten, bevorzugt Dentalimplantaten, vorgesehen. Gerade bei der Verwendung des Hyaluronsäuregels im Zusammenhang mit einem Dentalimplantat kann das Hyaluronsäuregel in die parodontale Weichgewebetasche eingegeben, bevorzugt eingespritzt, werden. In dieser parodontalen Weichgewebetasche kann die Stabilität insbesondere dadurch gewährleistet werden, dass die Hyaluronsäure mit dem dort vorhandenen körpereigenen Blut binnen kurzer Zeit ein Koagulum bildet, so dass sie nicht mehr durch den im Mundraum vorhandenen Speichel ausgewaschen werden kann.

Die mit der Erfindung erzielten Vorteile bestehen insbesondere darin, dass durch die Einbettung der bioziden und damit infektionshemmenden Metallpartikel in die umhüllenden Gelpartikel in der Art einer Depotbildung der Wirkstoff geeignet im Körper hinterlegt werden kann und erst langsam und nach gewisser Verzögerungszeit dosiert freigesetzt wird. Damit kann auch über vergleichsweise lange Zeiträume hinweg eine zuverlässige infektionshemmende Wirkungsweise sichergestellt werden. Durch die Verwendung von quervernetzter Hyaluronsäure als Umhüllung für die Metallpartikel ist dabei sichergestellt, dass ein zuverlässiger Abbau des Hüllmaterials stattfindet, wobei aufgrund der hohen Biokompatibilität der Hyaluronsäure nicht mit weiteren Beeinträchtigungen des Körpergewebes zu rechnen ist.

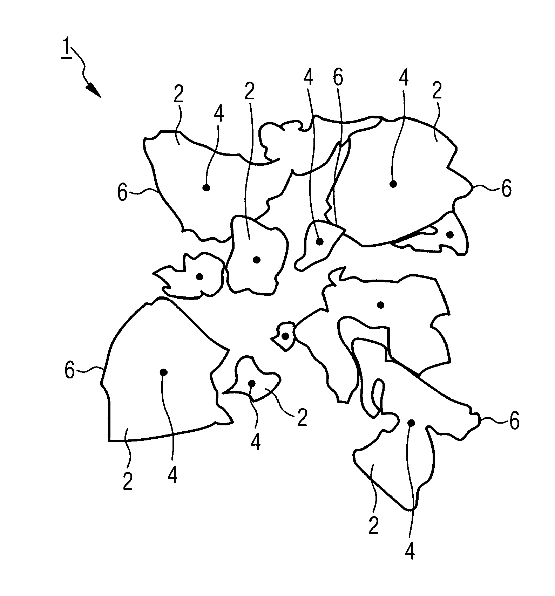

Ein Ausführungsbeispiel der Erfindung wird anhand einer Zeichnung näher erläutert. Darin zeigt die Figur schematisch ein Hyaluronsäuregel, gebildet aus Gelpartikeln.

Das Hyaluronsäuregel 1 gemäß der Figur umfasst von quervernetzter Hyaluronsäure gebildete Gelpartikel 2, in die Partikel 4 eines bioziden Metalls, im Ausführungsbeispiel Silber, eingebettet sind. Die Gelpartikel 2 weisen dabei im Ausführungsbeispiel eine mittlere Partikelgröße von etwa 150 µm auf und umhüllen die eingebetteten Silberpartikel 4 mit ihrer äußeren Hülle 6 vollständig. Die Silberartikel 4 weisen eine mittlere Partikelgröße von etwa 10 µm auf. Insbesondere kommt auch die Verwendung von kolloidalem Silber zur Bildung der Partikel 4 in Betracht, von dessen Wirksamkeit bei der Infektionsbekämpfung man seit geraumer Zeit ausgeht.

Zur Herstellung des Hyaluronsäuregels 1 wird zunächst ein Vordispergieren der Silberpartikel 4 in Glycerin als Dispergiermittel vorgenommen. Die dabei entstehende Dispersion oder Suspension der Silberpartikel 4 im Glycerin wird anschließend vorübergehend kontinuierlich in Bewegung gehalten, z. B. durch Rühren, so dass ein Sedimentieren vermieden wird. Diese Suspension oder Dispersion wird sodann in geeigneten Mengenverhältnissen mit der Ausgangslösung der Hyaluronsäure vermischt, so dass die gewünschte Suspension der Silberpartikel 4 in der Hyaluronsäure entsteht. Die Mengenverhältnisse werden dabei derart geeignet gewählt, dass unter Berücksichtigung der weiteren Bestandteile im Endprodukt, also im Hyaluronsäuregel 1, ein Gewichtsanteil der Silberpartikel 4 von etwa 0,3 % vorliegt.

Nach sorgfältiger Durchmischung der Komponenten Hyaluronsäure und Partikel 4, die eine weitgehend gleichmäßige Verteilung der Partikel 4 in der Ausgangslösung der Hyaluronsäure bewirkt, wird die Quervernetzung in Gang gesetzt. Dazu wird als Vernetzungsmittel 1,4-Butandioldiglycidylether (BDDE) zugegeben. Die Zugabe des Vernetzungsmittels erfolgt dabei ausreichend zeitnah nach Herstellung der Suspension, so dass die Partikel 4 sich nicht wieder absetzen können. Beim Prozess der Quervernetzung erfolgt der Einschluss der Partikel 4 in die sich infolge der Quervernetzung bildenden Gelpartikel 2. Durch geeignete Prozessführung, also insbesondere durch eine geeignete Wahl von Reaktionszeiten und -parametern wie beispielsweise Druck, Temperatur, Mischungsverhältnissen und dergleichen, wird dabei die Vernetzung der Hyaluronsäure derart durchgeführt, dass sich ein Vernetzungsgrad von höchstens etwa 10 % einstellt.

Nachdem die Quervernetzung der Hyaluronsäure mit Einschluss der Silberpartikel 4 in die entstehenden Gelpartikel 2 durchgeführt wurde, wird im Ausführungsbeispiel ein biphasisches Gel hergestellt, indem als weitere Komponente noch unvernetzte Hyaluronsäure zugemischt wird.

Anschließend oder bedarfsweise auch als Zwischenschritt wird das erhaltene Zwischenprodukt mit den entstandenen Partikeln 2 aus quervernetzter Hyaluronsäure mit darin eingebetteten Silberpartikeln 4 im Ausführungsbeispiel an sich üblichen Schritten der weiteren Behandlung unterzogen. Dabei ist ein Quellvorgang vorgesehen, und das nach der Vernetzung erhaltene Hyaluronsäuregel 1 wird gefiltert und gewaschen.

Die eingesetzte Hyaluronsäure weist im Ausführungsbeispiel ein Molekulargewicht von 500.000 bis 1.100.000 atomaren Masseneinheiten auf.

### Bezugszeichenliste

- 1: Hyaluronsäuregel
- 2: Gelpartikel
- 4: Partikel
- 6: Hülle

## Patentansprüche

1. Hyaluronsäuregel (1) mit aus quervernetzter Hyaluronsäure gebildeten Gelpartikeln (2), in die Partikel (4) eines bioziden Metalls eingebettet sind.

2. Hyaluronsäuregel (1) nach Anspruch 1, bei dem als biozides Metall zur Bildung der Partikel (4) Silber oder Kupfer vorgesehen ist.

3. Hyaluronsäuregel (1) nach Anspruch 1 oder 2, bei dem die Partikel (4) des bioziden Metalls eine mittlere Partikelgröße von etwa 0,2 µm bis 20 µm, vorzugsweise von etwa 10 µm, aufweisen.

4. Hyaluronsäuregel (1) nach einem der Ansprüche 1 bis 3, bei dem die die Gelpartikel (2) bildende Hyaluronsäure einen Vernetzungsgrad von höchstens 65 %, vorzugsweise von höchstens 10 %, aufweist.

5. Hyaluronsäuregel (1) nach einem der Ansprüche 1 bis 4, bei dem die Gelpartikel (2) eine mittlere Partikelgröße von etwa 50 µm bis etwa 600 µm, vorzugsweise von etwa 150 µm bis höchstens 300 µm, aufweisen.

6. Hyaluronsäuregel (1) nach einem der Ansprüche 1 bis 5, das als biphasisches Gel ausgeführt ist, wobei zusätzlich zu einem ersten, die Gelpartikel (2) enthaltenden Gelanteil ein zweiter Gelanteil, umfassend unvernetzte Hyaluronsäure, vorgesehen ist.

7. Hyaluronsäuregel (1) nach einem der Ansprüche 1 bis 6, in dem das biozide Metall mit einem Gewichtsanteil von 0,1 % bis 0,5 %, vorzugsweise von höchstens 0,3 %, vorliegt.

8. Verfahren zur Herstellung eines Hyaluronsäuregels (1), insbesondere nach einem der Ansprüche 1 bis 7, bei dem
- Hyaluronsäure mit Partikeln (4) eines bioziden Metalls versetzt wird, so dass sich eine Suspension bildet, und
- der Suspension ein Vernetzungsmittel für die Hyaluronsäure zugegeben wird, so dass die Hyaluronsäure quervernetzt wird.

9. Verfahren nach Anspruch 8, bei dem das nach der Vernetzung erhaltene Hyaluronsäuregel (1) gefiltert und gewaschen wird.

10. Verfahren nach Anspruch 8 oder 9, bei dem die Vernetzung der Hyaluronsäure derart durchgeführt wird, dass sich ein Vernetzungsgrad von höchstens 65%, besonders bevorzugt von höchstens 10 %, einstellt.

11. Verfahren nach einem der Ansprüche 8 bis 10, bei dem als Vernetzungsmittel 1,2-Bis(2,3-Epoxypropoxy)ethylen oder 1-(2,3-Epoxypropyl)-2,3-epoxycyclohexan oder besonders bevorzugt 1,4-Butandioldiglycidylether (BDDE) verwendet wird.

12. Verfahren nach einem der Ansprüche 8 bis 10, bei dem die Vernetzung der Hyaluronsäure durch UV-Licht-induzierte Quervernetzung durchgeführt wird.

13. Verfahren nach einem der Ansprüche 8 bis 12, bei dem die eingesetzte Hyaluronsäure ein Molekulargewicht von 300.000 bis 2.500.000, bevorzugt von 400.000 bis 1.500.000, besonders bevorzugt von 500.000 bis 1.100.000, atomaren Masseneinheiten aufweist.

14. Hyaluronsäuregel (1), erhältlich durch das Verfahren nach einem der Ansprüche 8 bis 13.

15. Verwendung eines Hyaluronsäuregels (1) nach einem der Ansprüche 1 bis 7 oder 14 in einem kosmetischen Mittel oder in einem Medizinprodukt.

16. Medizinprodukt, enthaltend ein Hyaluronsäuregel (1) nach einem der Ansprüche 1 bis 7 oder 14.

17. Medizinprodukt nach Anspruch 16 zur Schleimhaut- oder Wundbehandlung.

18. Medizinprodukt nach Anspruch 16 zur Behandlung und/oder Prophylaxe von Parodontose oder Periimplantitis.
